# FASCICULE DE BREVET EUROPEEN

(11) **EP 0 782 703 B1**
(45) Date de publication et mention de la délivrance du brevet: **23.06.1999**
(21) Numéro de dépôt: 95932046.6
(22) Date de dépôt: 22.09.1995
(51) Int. Cl.: G01N 33/52, A61B 10/00, C12Q 1/28

(54) **DISPOSITIF A USAGE UNIQUE DE DETECTION OU ANALYSE D'UN LIQUIDE CORPOREL**
EINWEG-VORRICHTUNG ZUM NACHWEIS ODER ZUR ANALYSE EINER KÖRPERFLÜSSIGKEIT
SINGLE-USE DEVICE FOR DETECTING OR ANALYZING A BODY FLUID

(30) Priorité: 22.09.1994 FR 9411547; 21.11.1994 FR 9414249
(43) Date de publication de la demande: 09.07.1997
(73) Titulaire: Chaffringeon, Bernard, 1025 Saint-Sulpice (CH)
(72) Inventeur: Chaffringeon, Bernard, 1025 Saint-Sulpice (CH)
(74) Mandataire: Guerre, Dominique
(86) Numéro de dépôt international: FR9501224
(87) Numéro de publication internationale: WO9609545

(56) Documents cités:
- EP-A- 0 166 933
- EP-A- 0 228 752
- EP-A- 0 363 196
- WO-A-91/09309
- US-A- 3 850 160
- US-A- 4 227 537
- US-A- 4 257 427
- US-A- 4 614 715

## Description

La présente invention concerne un dispositif à usage unique, d'analyse ou de détection d'un composant, ou d'une condition biologique ou biochimique d'un liquide corporel, présent dans une cavité intracorporelle allongée. A titre d'exemple non limitatif, la présente invention sera introduite, définie et expliquée par rapport à la détection de la période fertile de la femme, au moyen de la glaire cervicale prélevée dans sa cavité vaginale.

Afin de déterminer les périodes de fertilité de la femme, il est connu et il a été proposé de détecter et suivre la présence et/ou concentration en certains constituants biochimiques ou biologiques de ladite glaire, tels qu'une peroxydase ou un composé présentant une activité peroxydasique, ou tels qu'un mucopolysaccharide ou une glycoprotéine, par l'utilisation de réactifs ou systèmes réactifs colorés, par exemple, dans le premier cas un composé d'oxydoréduction, dont au moins la forme oxydée est colorée, par exemple le gaïacol, et dans le second cas la safranine.

Pour la mise en oeuvre de tels réactifs ou systèmes réactionnels, on a généralement proposé des solutions sommaires, et en pratique difficilement mises en oeuvre par la femme, à savoir :
- le prélèvement in situ de la glaire cervicale, par un dispositif susceptible de recueillir la glaire, tel qu'un écouvillon, puis la mise en contact dudit dispositif avec un réactif sous forme liquide ;
- l'introduction dans la cavité vaginale d'un tampon absorbant, imprégné ou enrobé d'une couche de réactif, et l'extraction dudit tampon au bout d'un certain temps, avec traitement de la couche de réactif par un développeur de coloration.

De telles solutions sont proposées par les documents FR-A-2 216 975, FR-A-2 399 231, EP-A-0 363 196, et EP-A-0 555 109.

Les inconvénients de ces méthodes résultent de la nécessité de traiter ensuite l'extrait de la glaire cervicale recueillie, avec des solutions séparées de développement de réaction colorée, afin de révéler la présence éventuelle ou concentration de certains de ses constituants biochimiques.

Par ailleurs, les différentes solutions jusqu'alors connues ne convenaient pas au recueil de glaire cervicale pour toutes les femmes, et ceci en raison des variations de fluidité et quantité de sécrétion de la glaire. En effet, les solutions précitées ne sont d'une utilité pratique que si les glaires sont présentes en relativement grande quantité ou sont très fluides.

Conformément au document US-C-4 257 427, on a décrit un dispositif à usage unique de détection d'un microorganisme présent dans une cavité intracorporelle. Ce dispositif comprend un élément de prélèvement allongé, creux, en matière plastique transparente, adapté pour être logé et retenu par simple constriction de la cavité intracorporelle, par l'intermédiaire d'un manchon souple en mousse. Ce même élément est donc intrinsèquement raide ou rigide selon sa longueur, pour être poussé par une extrémité et introduit par l'autre extrémité dans la cavité intracorporelle. Des moyens de prélèvement du liquide corporel présent dans la cavité intracorporelle consistent en une mèche, disposée de manière étanche dans le col de l'élément de prélèvement, comportant une extrémité extérieure effilochée, et une extrémité intérieure rassemblant le liquide corporel collecté. Un milieu de culture du microorganisme est disposé à l'intérieur de l'élément de prélèvement.

L'utilisateur introduit ce dispositif dans la cavité intracorporelle, le laisse y séjourner un temps suffisant pour cultiver in situ le microorganisme, puis extrait ledit dispositif afin d'observer ou non le développement d'une culture, au travers de la paroi de l'élément de prélèvement.

Un tel dispositif ne permet pas immédiatement, ou quasi-immédiatement l'analyse ou détection d'un composant ou d'une condition biologique ou biochimique.

La présente invention a pour objet un dispositif à usage unique, de détection extemporanée et directe d'un composant, ou d'une condition biologique ou biochimique d'un liquide corporel, présent en quantité et fluidité variables, tel que la glaire cervicale, dans une cavité intracorporelle allongée, telle que la cavité vaginale.

Plus particulièrement, la présente invention a pour objet un tel dispositif permettant la détection de la période fertile chez la femme à partir de glaires cervicales, lorsque celles-ci sont sécrétées en petites quantités ou sont relativement visqueuses, tout en demeurant particulièrement simple et confortable à utiliser.

Le dispositif selon l'invention comprend de manière générale, un capuchon complémentaire à l'élément de prélèvement allongé, comprenant des moyens réactionnels, distribués ou disposés sur ledit capuchon, notamment sur sa paroi latérale, susceptibles de réagir, de manière visible ou perceptible pour l'utilisateur, avec au moins un composant du liquide corporel, ou en présence d'une condition biologique ou biochimique dudit liquide, la forme et les dimensions du capuchon étant choisis pour mettre en contact les moyens de prélèvement de l'élément allongé et les moyens réactionnels du capuchon, notamment lors de ou après l'insertion du premier dans le second.

Les moyens de prélèvement aident au transfert de la glaire cervicale vers les moyens réactionnels du capuchon, ce qui permet à l'ensemble d'être utilisé d'une manière fiable et efficace par des femmes dont la sécrétion de glaires est minimale, ou dont la glaire est si visqueuse qu'elle ne permet pas l'utilisation de dispositifs de détection classiques. En effet, et d'une manière surprenante, il a été remarqué que l'ensemble selon l'invention convenait à la détection de la période fertile chez la femme, quelle que soit la viscosité des glaires cervicales sécrétées.

Préférentiellement, l'élément de prélèvement allongé est adapté en formes et dimensions, pour être logé et retenu dans la cavité intracorporelle, directement à son contact, et la surface extérieure est biocompatible au contact de ladite cavité intracorporelle.

Avantageusement, ces moyens consistent en des éléments filiformes ou fibres, en polymère synthétique ou naturel, biocompatible et hydrophile, choisi parmi les polyesters, les polyacrylonitriles, les polycarbonates, les polyéthylènes et polypropylènes, les silicones, les alginates, les mousses de polyuréthanne, ou encore la cellulose ou ses esters, de manière à assurer le transfert total de la glaire vers les moyens réactionnels. Dans certains cas, les éléments filiformes ou fibres peuvent être imprégnés avec un liquide favorisant la fluidité ou le transfert du liquide corporel.

Par "biocompatible", on entend le fait que le contact entre la surface extérieure de l'élément allongé et la paroi de la cavité intracorporelle ne génère aucune réaction biologique adverse, de type toxique ou allergique par exemple.

Le capuchon complémentaire est de préférence en matière plastique transparente ou translucide, et recouvre et entoure complètement une partie supérieure de l'élément de prélèvement allongé, par simple poussée de la main, de manière ajustée avec un jeu fonctionnel.

Les moyens reactionnels sont disposés à l'intérieur, sur au moins une paroi, par exemple la paroi latérale, du capuchon, et comprennent au moins un réactif, par exemple déposé sur une couche absorbante, susceptible de réagir avec au moins un composant du liquide corporel recueilli, pour donner au moins un produit de réaction, notamment coloré, révélant la présence dudit composant dans ledit liquide corporel.

L'élément allongé peut également comporter, si celui-ci est creux, du côté de son fond, un réactif comme décrit précédemment, identique ou différent du réactif du capuchon. Dans ce dernier cas, il est possible de détecter un autre composant ou une autre condition biologique ou biochimique du liquide corporel.

Préférentiellement, dans le cas d'un réactif disposé dans un élément allongé de prélèvement en forme de conduit, le fond de ce dernier est également transparent, et le réactif est disposé sur ce fond, et ceci en vis-à-vis de l'extrémité opposée à celle assurant le prélèvement du liquide corporel, de telle sorte que lorsque le produit de réaction est coloré, cette couleur ou absence de couleur peut être vue ou visualisée par l'utilisateur ou l'utilisatrice, directement lors du retrait du dispositif.

Avant l'utilisation, le capuchon repose sur, et entoure l'élément allongé de prélèvement. Lors de l'utilisation du dispositif, au moyen d'un applicateur standard tel qu'un applicateur de tampon, on enlève le capuchon. Après retrait de l'élément allongé, on replace aussitôt le capuchon sur ce dernier. Ainsi, on évite des pertes de liquide recueilli, tout en assurant une lecture rapide du résultat des moyens réactionnels, qui est visible à travers la matière transparente du capuchon.

L'ensemble de détection selon l'invention, décrit précédemment, peut donc être utilisé sans ajout ultérieur de réactif ou révélateur coloré par l'utilisateur ou l'utilisatrice.

La présente invention est maintenant décrite par référence au dessin annexé, dans lequel :
- la figure 1 représente une vue en coupe d'un dispositif à usage unique selon un premier mode d'exécution de l'invention ;
- la figure 2 représente une vue en coupe d'un moyen applicateur incorporant l'élément de prélèvement allongé selon l'invention ;
- la figure 3 représente un deuxième mode d'exécution d'un dispositif d'analyse selon l'invention ;
- la figure 4 représente l'élément de prélèvement du dispositif selon Fig.2, dans sa position déployée à l'intérieur de la cavité intracorporelle ;
- la figure 5 représente le capuchon du dispositif selon Fig.3, et la figure 6 l'intérieur du capuchon.

De manière générale, un dispositif 1 à usage unique, de détection extemporanée et directe d'un composant, ou d'une condition biologique ou biochimique d'un liquide corporel, dans une cavité intracorporelle allongée, dans laquelle ledit liquide corporel est présent, comprend :
- un élément 2 de prélèvement allongé, en forme d'élément de conduit, présentant deux extrémités, à savoir un col 2a et un fond 2b, adapté en forme et dimensions pour être logé et retenu par simple constriction de la cavité intracorporelle, directement à son contact ; cet élément est intrinsèquement et suffisamment raide ou rigide selon sa longueur, pour être poussé par son extrémité ou fond 2b, et introduit par l'autre extrémité ou col 2a dans la cavité intracorporelle ; des moyens 3 de prélèvement du liquide corporel, par contact dans la cavité intracorporelle, sont distribués ou disposés à l'extérieur de l'élément allongé 2, notamment sur tout son pourtour, et ;
- un capuchon 7 complémentaire à l'élément de prélèvement allongé 2, séparable de ce dernier, comprenant des moyens réactionnels 8, distribués ou disposés à l'intérieur dudit capuchon 7, notamment sur sa paroi latérale, susceptibles de réagir, de manière visible ou perceptible pour l'utilisateur, avec au moins un composant du liquide corporel, ou en présence d'une condition biologique ou biochimique dudit liquide, la forme et les dimensions du capuchon 7 étant choisis pour mettre en contact les moyens de prélèvement 3 de l'élément allongé 2 et les moyens réactionnels 8 du capuchon 7, lors ou après l'insertion du premier dans le second.

L'élément allongé de prélèvement 2 peut avoir une structure composite et comprendre un tube 5 relativement rigide, par exemple en matière plastique transparente, avec un col 5a et un fond 5b fermé par une paroi de fond 10, sur lequel est éventuellement emmanché un manchon 6 biocompatible, entourant au moins la paroi latérale du tube, constitué par exemple par un tube extérieur 6a en composés d'origine naturelle tels que la cellulose ou la ouate, et une matrice intérieure 6b rigide de support en polymère pour ledit deuxième tube, dont la bordure antérieure (dans le sens de l'introduction de l'ensemble) est arrondie, et dont la bordure postérieure est à bord droit.

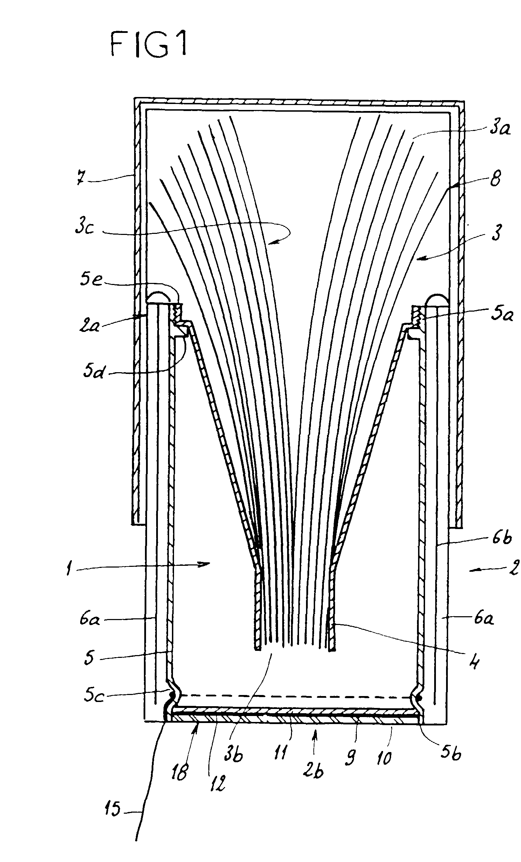

Les moyens de prélèvement 3 sont maintenus dans le col 2a,5a de l'élément 2, et disposés ou distribués à l'extérieur dudit élément ; de préférence et comme illustré à la figure 1, ils sont montés sur un moyen de support 4 amovible ou fixe, par rapport à l'élément allongé 2. Plus précisément, le moyen de support 4 a une forme d'entonnoir et est monté au tube rigide 5, grâce à un pas de vis 5e et un épaulement 5d prévus sur le tube 5, au niveau de son col 5a.

Les moyens de prélèvement 3 consistent en des éléments filiformes 3c disposés en gerbe, s'étendant d'une première extrémité 3a située à l'extérieur de l'élément allongé 2, du côté de son bord antérieur, à une deuxième extrémité 3b située à l'intérieur dudit élément allongé 2. Les moyens de prélèvement assurent ainsi le prélèvement du liquide corporel visqueux recueilli à l'extrémité 3a, et aussi son transfert vers l'extrémité 3b et les moyens réactionnels 18 qui seront décrits plus loin.

Les éléments filiformes 3c peuvent être remplacés par d'autres moyens ou matières (telles que mèches), assurant un transfert par absorption et/ou action capillaire du liquide ou fluide corporel collecté. Toutefois, l'avantage d'utiliser des filaments réside dans le fait que ces derniers se plient, et peuvent frotter au contact de la paroi intracorporelle de la cavité, lors de l'insertion de l'élément allongé 2 dans celle-ci, en prélevant ainsi plus de liquide ou fluide que si ce dernier était prélevé par simple écoulement par gravité.

Préférentiellement, on choisit des fibres hydrophiles, car celles-ci permettent un transfert par absorption ou par action capillaire de la glaire cervicale, qui est constituée pour sa grande partie par de l'eau, et par conséquent assurent un transfert plus complet vers les moyens réactionnels. Avantageusement, les éléments filiformes 3c consistent en des filaments ou fibres de polymères biocompatibles tels que les polyesters, les polyacrylonitriles, les polycarbonates, les polyéthylènes et polypropylènes, les silicones, les alginates, ou encore la cellulose ou ses esters.

Des moyens réactionnels 18 sont disposés dans l'élément allongé 2 du côté de son fond 2b, en vis-à-vis des moyens de prélèvement 3, et plus précisément de la deuxième extrémité 3b de ces derniers. Ces moyens réactionnels comprennent au moins un réactif 9, déposé sur une couche absorbante 12, susceptible de réagir avec au moins un composant du liquide corporel, ou en présence d'une condition biologique ou biochimique du liquide, pour donner un produit de réaction coloré, révélant la présence de ladite condition ou dudit composant.

Un lien d'extraction 15, sous la forme d'un fil ou cordon est fixé à l'élément de prélèvement allongé 2, du côté de l'extrémité 2b opposée à l'extrémité d'insertion, par l'intermédiaire d'une gorge 5c prévue dans le tube 5.

Le capuchon 7 présente une forme semblable à celle de l'élément de prélèvement 2, en l'occurrence cylindrique, avec des dimensions légèrement supérieures à ce dernier, de sorte que celui-ci s'ajuste étroitement dans la partie supérieure de l'élément 2, en frottant à l'intérieur contre les moyens de prélèvement 3. Le capuchon 7 se fixe donc sur l'élément de prélèvement 2 par simple ajustement, avec du jeu fonctionnel, par simple poussée de la main ou du doigt.

Des moyens réactionnels 8 sont disposés sur au moins une paroi, par exemple la paroi latérale, et à l'intérieur du capuchon 7.

Lorsqu'on prévoit deux moyens réactionnels 8, 18, les réactifs respectifs peuvent être identiques ou différents, pour permettre la détection de composants ou conditions biologiques, identiques ou différents. Les moyens réactionnels 18 incorporent au moins un réactif 9, et éventuellement un agent fluidifiant pour la glaire cervicale, déposé par exemple sur une couche absorbante 12 contre la paroi de fond transparente 10 du tube 5, susceptible de réagir avec au moins un composant du liquide corporel recueilli, pour donner au moins un produit de réaction coloré, révélant la présence dudit composant dans le liquide corporel.

De préférence, lorsque le composant dont on recherche la présence est un composant présentant une activité peroxydasique ou pseudoperoxydasique, les moyens réactionnels 8, 18 comprennent un composé d'oxydoréduction, dont au moins la forme oxydée est colorée, par exemple le gaïacol.

A titre d'exemple, pour la détection d'une glycoprotéine ou d'un mucopolysaccharide, et par conséquent, la période fertile chez la femme, les moyens réactionnels peuvent comprendre les réactifs suivants : la safranine, le bleu de toluidine O, le bleu Alcian, le bleu trypan, un sel de tolonium, le PAS (Schiff périodique acide) ou un mélange de ceux-ci, éventuellement couplés à un agent favorisant la réaction colorée, tel que le polyvinylpyrrolidone.

La couche 12 imprégnée du réactif 9 peut être recouverte par un voile 11 hemi-perméable, en ce sens qu'il est perméable vis-à-vis du liquide corporel transféré, dans le sens du passage de ce dernier à partir des moyens de prélèvement 3, mais qu'il retient ce même liquide dans l'autre sens. Ce voile peut également être imprégné de l'agent fluidifiant pour la glaire cervicale, ledit agent favorisant le passage des composés que l'on souhaite détecter et étant par exemple un agent tensioactif.

Le moyen réactionnel 8 du capuchon 7 peut être agencé de la même manière que le moyen réactionnel 18 de l'élément allongé de prélèvement 2.

Par ailleurs, les moyens de prélèvement 3 peuvent également être recouverts d'un agent fluidifiant, afin de faciliter le transfert du liquide corporel visqueux vers les moyens réactionnels 8.

De manière générale, mais comme non représenté à la figure 1, la forme et les dimensions de l'élément 2 de conduit sont adaptées à celles de la cavité vaginale.

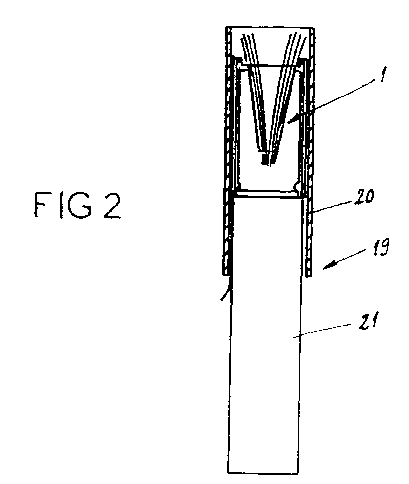

Par référence à la figure 2, le dispositif 1 précédemment décrit peut faire partie d'un système prêt à l'emploi comprenant ce dispositif 1 et des moyens applicateurs 19. Ces moyens applicateurs 19 sont connus par l'homme du métier et comprennent en général un tube-guide 20, à l'intérieur duquel l'élément allongé de prélèvement 2 est inséré, après enlèvement et séparation du capuchon 7, et un poussoir 21 logé à l'intérieur du tube-guide, en butée contre l'élément allongé. Un tel système peut être introduit directement dans la cavité intracorporelle, par exemple la cavité vaginale, et en poussant le poussoir 21, on libère l'élément allongé par rapport au moyen applicateur 19, pour le mettre en place dans cette même cavité.

Après retrait de l'élément de prélèvement 2 de la cavité intracorporelle, l'utilisateur replace le capuchon 7 transparent. Les moyens réactionnels 8 prévus à l'intérieur de ce dernier viennent alors en contact avec les moyens de prélèvement 3. Dans le cas où les moyens de prélèvement 3 comportent des filaments ou fibres, ceux-ci frottent contre les moyens réactionnels 8 et déposent ainsi le liquide corporel recueilli sur la couche absorbante. De cette manière, l'utilisateur s'aperçoit immédiatement de l'existence d'une réaction colorée, et par conséquent du composant ou de la condition biologique ou biochimique recherchée dans le liquide corporel.

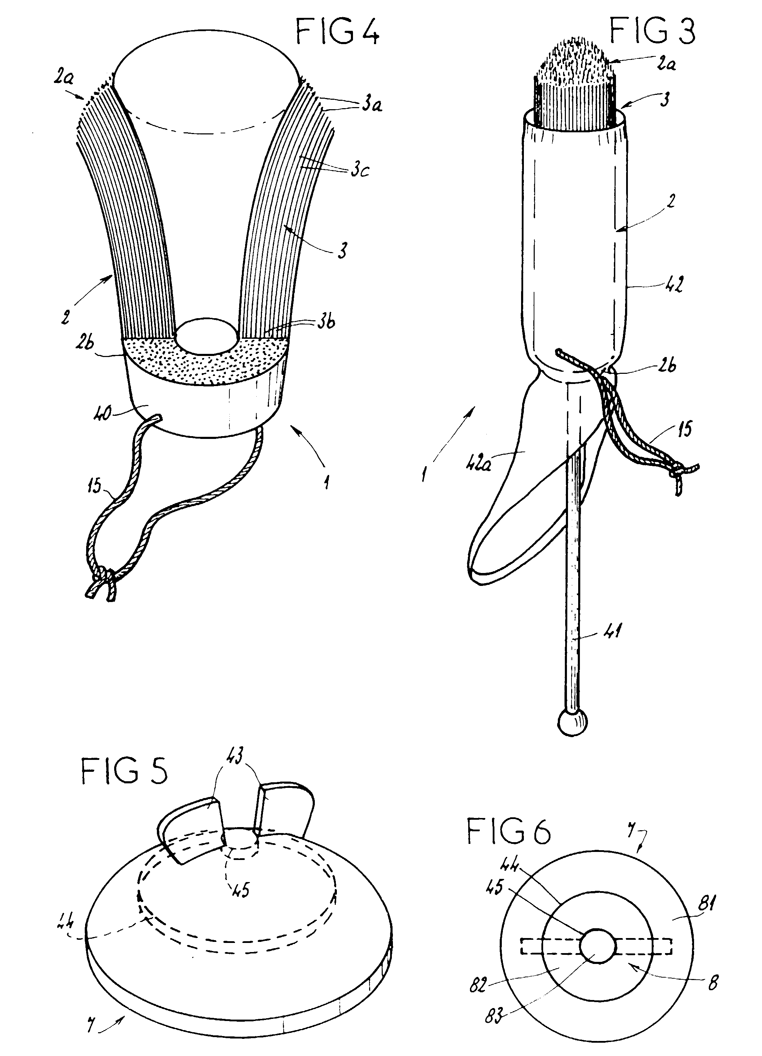

Le dispositif de détection selon Figures 3 à 6 diffère de celui précédemment décrit par référence aux figures 1 et 2, par les caractéristiques suivantes :
- l'élément allongé du prélèvement 2 a la forme générale d'un blaireau, et comprend un renfort 40 sur lequel est fixée une couronne d'éléments filiformes 3c formant le moyen 3 de prélèvement ;
- avant et pour l'introduction de l'élément de prélèvement 3 dans la cavité intracorporelle, une gaine 42 est disposée autour de la couronne précitée, notamment de manière à rassembler les éléments filiformes 3c sous la forme d'un cylindre ; une fois l'élément de prélèvement 2 dans la cavité intracorporelle, la gaine 42 peut être pelée vers le bas, et retirée, en tirant sur sa partie 42a en anse ;
- une tige 41 relativement rigide permet de pousser l'élément de prélèvement 2 ; cette tige peut ensuite être séparée de ce dernier ;
- le capuchon 7 a la forme d'une cupule adaptée à recouvrir la couronne des éléments filiformes 3c, dans leur position en corolle montrée à la figure 4 ; ce capuchon comporte à l'extérieur deux ergots 43 de préhension et rotation, et à l'intérieur 3 zones concentriques 81 à 83 de réactifs, le tout formant le moyen réactionnel 8.

Selon les stades du cycle menstruel, la glaire étant plus ou moins filante, les éléments filiformes 3c vont s'écarter vers la périphérie lorsque la glaire est très filante, étant donné qu'ils ne sont pas soumis à une force adhésive. Inversement, lorsque la glaire l'est moins et qu'elle est moins abondante, les éléments 3c de la partie centrale adhérant les uns aux autres, restent dans cette position et ne se déplacent pas vers la périphérie. Une simple investigation visuelle permet déjà de situer la période du cycle selon un mode d'emploi fourni avec le dispositif. Pour confirmer et préciser le stade, le dispositif retiré est appliqué sur le capuchon 7 contenant des réactifs colorés 81 à 83.

Ce capuchon 4 est compartimenté circulairement par des cloisons 45 de quelques millimètres de hauteur, pour permettre aux extrémités des éléments 3c de rester dans la position qu'ils avaient à l'intérieur de la cavité intracorporelle et au retrait de l'élément 2. En faisant tourner l'élément 2 au contact du fond du capuchon, on obtient un ensemble de réactions biochimiques colorées, qui sont à comparer à une charte des différentes réactions colorées attendues, fournie avec le dispositif et commentée.

Un dispositif de détection selon l'invention peut être utilisé, non seulement pour la détection d'un composant dans un liquide corporel, mais aussi pour détecter toute condition chimique, biochimique ou biologique à partir d'un prélèvement de ce même liquide corporel, de cellules, ou de tissu biologique, à des fins de diagnostic, prophylactiques ou thérapeutiques. Ceci signifie que ce dispositif de détection peut incorporer des réactifs ou systèmes réactionnels très différents, de type purement chimique, par exemple enzymatique, ou biologique, par exemple un antigène ou un anticorps.

En conséquence, un dispositif de détection ou analyse selon l'invention peut recevoir de très larges applications, au rang desquelles on peut citer :
- la détection d'une hormone, et notamment d'un pic hormonal ;
- le prélèvement et l'analyse histologique et/ou cytochimique d'un liquide corporel, notamment pour détecter des états pathologiques, ou mettre en évidence certaines périodes physiologiques d'un cycle naturel, par exemple d'un cycle hormonal.

## Revendications

1. Dispositif (1) à usage unique d'analyse ou détection d'un composant, ou d'une condition biologique ou biochimique d'un liquide corporel présent dans une cavité intracorporelle en quantité et fluidité variables, ledit dispositif comprenant un élément (2) de prélèvement allongé, adapté pour être logé et retenu par simple constriction de la cavité intracorporelle, intrinsèquement et suffisamment raidie ou rigide selon sa longueur pour être poussé par une extrémité (2b) et introduit par l'autre extrémité (2a) dans la cavité intracorporelle, avec des moyens (3) de prélèvement de liquide corporel, distribués ou disposés à l'extérieur de l'élément allongé, **caractérisé en ce qu'il comprend** un capuchon (7) complémentaire à l'élément allongé (2), séparable de ce dernier, comprenant des moyens réactionnels (8), distribués ou disposés sur ledit capuchon (7), notamment sur sa paroi latérale, susceptibles de réagir, de manière visible ou perceptible pour l'utilisateur, avec au moins un composant du liquide corporel, ou en présence d'une condition biologique ou biochimique dudit liquide, la forme et les dimensions dudit capuchon étant choisis pour mettre en contact les moyens de prélèvement (3) de l'élément allongé (2) et les moyens réactionnels (8) du capuchon (7), notamment lors ou après insertion du premier dans le second.

2. Dispositif selon la revendication 1, caractérisé en ce que l'élément (2) de prélèvement allongé est adapté en forme et dimensions pour être logé et retenu dans la cavité intracorporelle, directement à son contact, et la surface extérieure est biocompatible au contact de ladite cavité intracorporelle.

3. Dispositif selon la revendication 2, caractérisé en ce que l'élément allongé (2) comprend un tube (5) relativement rigide, avec un col (5a), dans lequel sont disposés les moyens (3) de prélèvement, et un fond (5b), et éventuellement un manchon (6) biocompatible entourant au moins la paroi latérale dudit tube.

4. Dispositif selon la revendication 1, caractérisé en ce que les moyens de prélèvement (3) sont montés sur un moyen de support (4) amovible ou fixe par rapport à l'élément allongé (2).

5. Dispositif selon la revendication 1, caractérisé en ce que les moyens de prélèvement (3) consistent en des éléments filiformes (3c) ou fibres, en polymère synthétique ou naturel, biocompatible et hydrophile, choisi parmi les polyesters, les polyacrylonitriles, les polycarbonates, les polyéthylènes et polypropylènes, les silicones, les alginates, ou encore la cellulose ou ses esters.

6. Dispositif selon la revendication 1, caractérisé en ce que le capuchon 7 complémentaire est en matière plastique transparente.

7. Dispositif selon la revendication 1, caractérisé en ce que les moyens (3) de prélèvement sont distribués sur le pourtour de l'élément (2) de prélèvement, et les moyens réactionnels (8) sont distribués ou disposés à l'intérieur, notamment sur la paroi latérale dudit capuchon (7).

8. Dispositif selon les revendications 1 à 3, caractérisé en ce que des moyens réactionnels (18) sont également disposés dans l'élément allongé (2), du côté de son fond (2b), en vis-à-vis des moyens de prélèvement (3), et comprennent au moins un réactif (9), éventuellement déposé sur une couche absorbante (12), susceptible de réagir avec au moins un composant du liquide corporel, ou en présence d'une condition biologique ou biochimique dudit liquide, pour donner au moins un produit de réaction, notamment coloré, révélant la présence de ladite condition biologique ou biochimique, ou dudit composant dans ledit liquide corporel.

9. Dispositif selon les revendications 7 et 8, caractérisé en ce que le produit de réaction est coloré, la paroi du capuchon (7), et le fond (2b) de l'élément allongé (2), sont transparents (10), et le réactif (9) est disposé en couche relativement mince sur ladite paroi et ledit fond, en vis-à-vis des moyens (3) de prélèvement.

10. Dispositif selon la revendication 9, caractérisé en ce que la couche de réactif (9) est recouverte par un voile (11) hemi-perméable, perméable vis-à-vis du liquide corporel, dans le sens du passage de ce dernier à partir des moyens de prélèvement (3), et retenant ledit liquide dans l'autre sens, et en ce que le voile ou la couche de réactif ou les deux sont éventuellement imprégnés d'un agent fluidifiant favorisant le passage des composants à détecter.

11. Dispositif selon la revendication 1, caractérisé en ce que la forme et les dimensions de l'élément (2) de prélèvement sont adaptées à celles de la cavité vaginale, et le liquide corporel comprend de la glaire cervicale.

12. Dispositif selon la revendication 10, caractérisé en ce que le composant de la glaire cervicale dont on recherche la présence est un composant présentant une activité peroxydasique ou pseudoperoxydasique, et les moyens réactionnels (8) comprennent un composé d'oxydoréduction, dont au moins la forme oxydée est colorée, par exemple le gaïacol.

13. Dispositif selon la revendication 10, caractérisé en ce que le composant de la glaire cervicale dont on recherche la présence est un mucopolysaccharide ou une glycoprotéine, et les moyens réactionnels (8) comprennent un composé choisi parmi la safranine, le bleu de toluidine O, le bleu Alcian, le bleu trypan, un sel de tolonium, le PAS (Schiff périodique acide), la phosphatase alcaline ou un mélange de ceux-ci.

14. Ensemble prêt à l'emploi, comprenant un dispositif (1) selon la revendication 1 et un moyen applicateur (19).

## Patentansprüche

1. Vorrichtung (1) zur einmaligen Verwendung zum Analysieren oder Aufspüren eines Bestandteils oder einer biologischen oder biochemischen Beschaffenheit einer Körperflüssigkeit, die sich in veränderlicher Menge und Fließfähigkeit in einer Körperhöhle befindet, wobei die Vorrichtung ein längliches Entnahmeelement (2) aufweist, das geeignet ist, durch einfache Kontraktion der Körperhöhle aufgenommen und gehalten zu werden, und das über seine Länge in sich starr und fest genug ist, um an einem Ende (2b) geschoben und mit dem anderen Ende (2a) in die Körperhöhle eingeführt zu werden, mit Mitteln (3) zum Entnehmen der Körperflüssigkeit, die außerhalb des länglichen Elements verteilt oder angeordnet sind, dadurch gekennzeichnet, daß die Vorrichtung (1) eine zu dem länglichen Element (2) komplementäre Kappe (7) aufweist, die von letzterem abnehmbar ist und die Reaktionsmittel (8) aufweist, welche auf der Kappe (7), insbesondere auf ihrer Seitenwand, verteilt oder angeordnet sind und welche in der Lage sind, in einer für den Anwender sichtbaren oder wahrnehmbaren Art mit zumindest einem Bestandteil der Körperflüssigkeit oder bei Vorliegen einer biologischen oder biochemischen Beschaffenheit der Flüssigkeit zu reagieren, wobei die Form und die Abmessungen der Kappe derart gewählt sind, daß die Entnahmemittel (3) des länglichen Elements (2) und die Reaktionsmittel (8) der Kappe (7) in Kontakt kommen, und zwar insbesondere bei oder nach dem Einsetzen des Ersten in das Zweite.

2. Vorrichtung nach Anspruch 1, dadurch gekennzeichnet, daß das längliche Entnahmeelement (2) in seiner Form und seinen Abmessungen daran angepaßt ist, in direktem Kontakt in der Körperhöhle aufgenommen und gehalten zu werden, und daß die Außenseite in Kontakt mit der Körperhöhle biologisch verträglich ist.

3. Vorrichtung nach Anspruch 2, dadurch gekennzeichnet, daß das längliche Element (2) eine relativ feste Röhre (5) aufweist, mit einem Hals (5a), in dem die Entnahmemittel (3) angeordnet sind, und mit einem Boden (5b) und ggf. einer biologisch verträglichen Manschette (6), die wenigstens die Seitenwand der Röhre umgibt.

4. Vorrichtung nach Anspruch 1, dadurch gekennzeichnet, daß die Entnahmemittel (3) auf einem in bezug auf das längliche Element (2) unbeweglichen bzw. festen Haltemittel (4) angeordnet sind.

5. Vorrichtung nach Anspruch 1, dadurch gekennzeichnet, daß die Entnahmemittel (3) fadenförmige Elemente (3c) oder Fasern aus synthetischem oder natürlichem Polymer beinhalten, die biologisch verträglich und hydrophil sind und die ausgewählt sind aus Polyester, Polyacrylnitril, Polycarbonat, Polyethylen und Polypropylen, Silikonalginat und außerdem Zellulose oder seine Ester.

6. Vorrichtung nach Anspruch 1, dadurch gekennzeichnet, daß die komplementäre Kappe (7) aus einem durchsichtigen Plastikmaterial besteht.

7. Vorrichtung nach Anspruch 1, dadurch gekennzeichnet, daß die Entnahmemittel (3) an dem Rand des Entnahmeelements (2) verteilt sind und daß die Reaktionsmittel (8) im Inneren verteilt bzw. angeordnet sind, und zwar vorzugsweise auf der Seitenwand der Kappe (7).

8. Vorrichtung nach einem der Ansprüche 1 bis 3, dadurch gekennzeichnet, daß in dem länglichen Element (2), und zwar in der Nähe seines Bodens (2b) und gegenüber von den Entnahmemitteln (3), gleichermaßen Reaktionsmittel (18) angeordnet sind, die zumindest ein Reagenz (9) aufweisen, unter Umständen auf einer absorbierenden Schicht (12) angeordnet, das in der Lage ist, mit zumindest einem Bestandteil der Körperflüssigkeit oder bei Vorliegen einer biologischen oder biochemischen Beschaffenheit der Flüssigkeit zu reagieren, um zumindest ein Reaktionsprodukt zu ergeben, und zwar vorzugsweise farbig, das die Anwesenheit der biologischen oder biochemischen Beschaffenheit oder des Bestandteils in der Körperflüssigkeit anzeigt.

9. Vorrichtung nach Anspruch 7 und 8, dadurch gekennzeichnet, daß das Reaktionsprodukt farbig ist, daß die Wand der Kappe (7) und der Boden (2b) des länglichen Elements (2) transparent (10) sind und daß das Reagenz (9) in einer relativ dünnen Schicht auf der Wand und dem Boden angeordnet ist, und zwar gegenüber von den Entnahmemitteln (3).

10. Vorrichtung nach Anspruch 9, dadurch gekennzeichnet, daß die Schicht des Reagenz (9) von einer semipermeablen Abdekkung (11) bedeckt ist, die bezüglich der Körperflüssigkeit in' der Richtung ihres Durchgangs vom Anfang der Entnahmemittel (3) durchlässig ist und die die Flüssigkeit in der anderen Richtung zurückhält, und daß die Abdeckung oder die Schicht des Reagenz oder beide unter Umständen mit einem Verflüssigungsmittel getränkt sind, das den Durchgang der aufzuspürenden Bestandteile begünstigt.

11. Vorrichtung nach Anspruch 1, dadurch gekennzeichnet, daß die Form und die Abmessungen des Entnahmeelementes (2) angepaßt sind an diejenigen der Vaginalhöhle und daß die Körperflüssigkeit Cervikalschleim aufweist.

12. Vorrichtung nach Anspruch 11, dadurch gekennzeichnet, daß der Bestandteil des Cervikalschleims, nach dessen Anwesenheit man sucht, ein Bestandteil ist, der eine peroxidasische oder pseudoperoxidasische Wirkung zeigt, und daß die Reaktionsmittel (8) eine Redoxverbindung aufweisen, bei der zumindest die oxidierte Form gefärbt ist, z.B. Guajakol.

13. Vorrichtung nach Anspruch 11, dadurch gekennzeichnet, daß der Bestandteil des Cervikalschleims, nach dessen Anwesenheit man sucht, ein Mucopolysaccharid oder Glykoprotein ist und daß die Reaktionsmittel (8) einen Bestandteil aufweisen, der aus Safranin, Toluidin-Blau O, Alcian-Blau, Trypan-Blau, einem Toloniumsalz, PAS (Periodic Acid-Schiff), alkalischer Phosphatase oder einem Gemisch davon ausgewählt ist.

14. Gebrauchsfertigter Zusammenbau mit einer Vorrichtung (1) nach Anspruch 1 und einem Applikationsmittel (19).

## Claims

1. Disposable device (1) for analysis or detection of a component, or of a biological or biochemical state, of a body fluid present in an elongate intracorporeal cavity in variable quantity and fluidity, the said device comprising an elongate sampling element (2), adapted to be fitted and held by simple constriction of the intracorporeal cavity, and inherently and sufficiently stiff or rigid along its length for it to be pushed via one end (2b) and introduced via the other end (2a) into the intracorporeal cavity, with means (3), for removing a sample of body fluid, which are distributed or arranged outside the elongate element, characterized in that the said device comprises a cap (7) complementary to the elongate element (2), and separable from the latter, having reaction means (8) distributed or arranged on the said cap (7), in particular on its side wall, which are capable of reacting, in a manner perceptible or visible to the user, with at least one component of the body fluid, or in the presence of a biological or biochemical state of the said fluid, and the shape and the dimensions of the said cap being chosen in order to bring the sampling means (3) of the elongate element (2) and the reaction means (8) of the cap (7) into contact, in particular during or after insertion of the former into the latter.

2. Device according to Claim 1, characterized in that the elongate sampling element (2) is adapted in shape and dimensions to be fitted and held in the intracorporeal cavity, directly in contact therewith, and the outer surface is biocompatible on contact with the said intracorporeal cavity.

3. Device according to Claim 2, characterized in that the elongate element (2) comprises a relatively rigid tube (5), with a neck (5a) in which the sampling means (3) are arranged, and a base (5b), and if appropriate a biocompatible (6) sleeve surrounding at least the side wall of the said tube.

4. Device according to Claim 1, characterized in that the sampling means (3) are mounted on a support means (4) which is removable or fixed in relation to the elongate element (2).

5. Device according to Claim 1, characerized in that the sampling means (3) consist of filiform elements (3c) or fibres made of biocompatible and hydrophilic, natural or synthetic polymer, chosen from among polyesters, polyacrylonitriles, polycarbonates, polyethylenes and polypropylenes, silicones, alginates, or else cellulose or its esters.

6. Device according to Claim 1, characterized in that the complementary cap 7 is made of transparent plastic.

7. Device according to Claim 1, characterized in that the sampling means (3) are distributed on the circumference of the sampling element (2), and the reaction means (8) are distributed or arranged on the inside, in particular on the side wall, of the said cap (7).

8. Device according to Claims 1 to 3, characterized in that reaction means (18) are also arranged in the elongate element (2), at its base (2b), facing the sampling means (3), and they comprise at least one reagent (9), if appropriate deposited on an absorbent layer (12), capable of reacting with at least one component of the body fluid, or in the presence of a biological or biochemical state of the said fluid, in order to give at least one reaction product, in particular coloured, revealing the presence of the said biological or biochemical state or of the said component in the said body fluid.

9. Device according to Claims 7 and 8, characterized in that the reaction product is coloured, the wall of the cap (7) and the base (2b) of the elongate element (2) are transparent (10), and the reagent (9) is arranged in a relatively thin layer on the said wall and the said base, facing the sampling means (3).

10. Device according to Claim 9, characterized in that the layer of reagent (9) is covered by a web (11) which is semipermeable, in the sense that it is permeable with respect to the body fluid, in the direction of the passage of the latter from the sampling means (3), while it holds back the said fluid in the other direction, and in that the web or the layer of reagent or both are impregnated if appropriate with a liquefying agent promoting the passage of the compounds which are to be detected.

11. Device according to Claim 1, characterized in that the shape and the dimensions of the sampling element (2) are adapted to those of the vaginal cavity, and the body fluid comprises cervical mucus.

12. Device according to Claim 10, characterized in that the component of the cervical mucus whose presence is to be detected is a component having a peroxidase activity or pseudo-peroxidase activity, and the reaction means (8) comprise an oxidation-reduction compound, of which at least the oxidized form is colored, for example guaiacol.

13. Device according to Claim 10, characterized in that the component of the cervical mucus whose presence is to be detected is a mucopolysaccharide or a glycoprotein, and the reaction means (8) comprise a compound chosen from among safranin, toluidine blue 0, Alcian blue, trypan blue, a tolonium salt, PAS (periodic acid-Schiff), alkaline phosphatase or a mixture of these.

14. Assembly ready for use, comprising a device (1) according to Claim 1 and an applicator means (19).
